(19) Europäisches Patentamt
European Patent Office
Office européen des brevets

(11) Veröffentlichungsnummer: **0 250 505**
**B1**

(12) # EUROPÄISCHE PATENTSCHRIFT

(45) Veröffentlichungstag der Patentschrift: **04.07.90**

(21) Anmeldenummer: **87900109.7**

(22) Anmeldetag: **06.12.86**

(86) Internationale Anmeldenummer:
**PCT/EP86/00720**

(87) Internationale Veröffentlichungsnummer:
**WO 87/03870 02.07.87 Gazette 87/14**

(51) Int. Cl.$^5$: **C 07 C 331/04,**
C 07 D 213/36,
C 07 D 239/26, C 09 K 19/30,
C 09 K 19/34

(54) **FLÜSSIGKRISTALLINE ISOTHIOCYANATE-DERIVATE.**

(30) Priorität: **20.12.85 DE 3545345**

(43) Veröffentlichungstag der Anmeldung:
**07.01.88 Patentblatt 88/01**

(45) Bekanntmachung des Hinweises auf die Patenterteilung:
**04.07.90 Patentblatt 90/27**

(84) Benannte Vertragsstaaten:
**CH DE FR GB IT LI NL SE**

(56) Entgegenhaltungen:
**EP-A-0 126 883**
**EP-A-0 167 912**
**EP-A-0 169 327**
**WO-A-86/03769**
**WO-A-86/04060**

**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **SCHEUBLE, Bernhard**
**Bluff 100 100-1, Yamate-cho Naka-ku,**
**Yokohama-shi**
**Kanagawa 231 (JP)**
Erfinder: **EIDENSCHINK, Rudolf**
**Konrad Adenauer Strasse 1**
**D-6109 Mühltal 1 (DE)**
Erfinder: **KRAUSE, Joachim**
**Samuel-Morse-Str. 14**
**D-6110 Dieburg (DE)**
Erfinder: **POETSCH, Eike**
**Am Buchwald 4**
**D-6109 Mühltal 6 (DE)**
Erfinder: **WÄCHTLER, Andreas**
**Goethestr. 34**
**D-6103 Griesheim (DE)**

(56) Entgegenhaltungen:
**Chemical Abstracts, Band 104, Nr. 18, 5. Mai 1986, Columbus, Ohio, USA, siehe Seite 721, Zusammenfassung 159748n**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 250 505 B1

**Beschreibung**

Die Erfindung betrifft Senföle der Formel I,

$$R—(A^1—Z^1)_n—A^2—Z^2—A^3—NCS \qquad (I)$$

worin

R: H oder Alkyl mit 1—15 C-Atomen, worin auch eine oder zwei nicht benachbarte $CH_2$-Gruppen durch —CH=CH—, —O—, —CO—, —O—CO— und/oder —CO—O— ersetzt sein können,

$A^1$ und $A^2$: jeweils eine unsubstituierte oder ein- oder mehrfach substituierte 1,4-Cyclohexylengruppe, eine Piperidin-1,4-diyl- oder 1,4-Bicyclo[2,2,2]octylengruppe oder eine unsubstituierte oder durch ein oder zwei F- und/oder Cl-Atome und/oder $CH_3$-Gruppen und/oder CN-Gruppen substituierte 1,4-Phenylengruppe, worin auch eine oder zwei CH-Gruppen durch N ersetzt sein können,

$A^3$: eine unsubstituierte oder ein- oder mehrfach substituierte 1,4-Cyclohexylengruppe oder eine unsubstituierte oder durch ein oder zwei F- und/oder Cl-Atome und/oder $CH_3$-Gruppen und/oder $CF_3$-Gruppen und/oder CN-Gruppen substituierte 1,4-Phenylgruppe,

$Z^1$ und $Z^2$: jeweils —CO—O—, —O—CO—, —O—, —$CH_2CH_2$—, —CHCN—$CH_2$—, —$CH_2$—CHCN—, —CH=CH—, —$OCH_2$—, —$CH_2O$—, —CH=N—, —N=CH—, —NO=N—, —N=NO— oder eine Einfachbindung,

n: 0, 1 oder 2 bedeutet,

mit der Maßgabe, daß, wenn $A^3$ eine unsubstituierte 1,4-Phenylengruppe bedeutet, n 2 ist oder $Z^2$ keine Einfachbindung oder $A^2—Z^2$ Pyr oder Cy—$CH_2CH_2$— bedeutet und im Falle $Z^2$=—CO—O— —$(A^1—Z^1)_n—A^2$— nicht

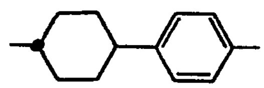

oder 1,4-Phenylen bedeutet.

Der Einfachheit halber bedeuten im folgenden Cy eine 1,4-Cyclohexylengruppe, Bi eine Bicyclo[2,2,2]-octylen-1,4-diylgruppe, Pip eine Piperidin-1,4-diylgruppe, Phe eine 1,4-Phenylengruppe, Pym eine Pyrimidin-2,5-diylgruppe, Pyr eine Pyridin-2,5-diylgruppe und Pzy eine Pyrazin-2,5-diylgruppe, wobei Cy und/oder Phe und/oder Pyr und/oder Pyz unsubstituiert oder durch ein oder zwei F- und/oder Cl-Atome und/ oder $CH_3$-Gruppen und/oder $CF_3$-Gruppen und/oder CN-Gruppen substituiert sein können.

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind und insbesondere eine vergleichsweise geringe Viskosität besitzen sowie eine verminderte Tendenz zur Bildung von Assoziaten aufweisen.

Aus der EP—O 126 883 sind flüssigkristalline Senföle bekannt, die eine trans-Cyclohexylphenylgruppe enthalten.

Diese Weisen jedoch vergleichsweise enge nematische Phasenbereiche auf.

Es wurde nun gefunden, daß Verbindungen der Formel I als Komponenten flüssigkristalliner Phasen vorzüglich geeignet sind. Insbesondere verfügen sie über vergleichsweise niedere Viskositäten und weisen keine oder nur eine geringe Tendenz zur Bildung molekularer Assoziate auf. Mit ihrer Hilfe lassen sich stabile flüssigkristalline Phasen mit breitem Mesophasenbereich und vorteilhaften Werten für die optische und dielektrische Anisotropie erhalten.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialen dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu erniedrigen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie ein Verfahren zur Herstellung der Verbindungen der Formel I nach Anspruch 1, dadurch gekennzeichnet, daß man ein entsprechendes Amin mit Thiophosgen behandelt, oder daß man ein entsprechendes Amin mit Schwefelkohlenstoff in ein Dithiocarbamat überführt und dieses mit Wasserstoffperoxid oder einem Chlorkohlensäureester behandelt, oder daß man ein entsprechendes Halogenid der Formel I, worin $A^3$ eine Cyclohexylengruppe bedeutet, mit einem Salz der Thiocyansäure umsetzt,

oder daß man eine Verbindung, die sonst der Formel I entspricht, aber an Stelle von H-Atomen eine oder mehrere reduzierbare Gruppen und/oder C—C-Bindungen enthält, mit einem reduzierenden Mittel behandelt,

oder daß man zur Herstellung von Verbindungen der Formel I, worin $A^3$ eine durch ein oder zwei F- und/oder Cl-Atome und/oder CN-Gruppen substituierte 1,4-Phenylengruppe bedeutet, in einem entsprechenden Diazoniumsalz die Diazoniumgruppe durch F, Cl oder CN ersetzt,

oder daß man zur Herstellung von Benzotrifluoriden der Formel I, worin $A^3$ eine durch eine oder zwei $CF_3$-Gruppen substituierte 1,4-Phenylengruppe bedeutet, eine entsprechende Carbonsäure oder deren Anhydrid mit Schwefeltetrafluorid umsetzt,

oder daß man zur Herstellung von Estern der Formel I (worin $Z^1$ und/oder $Z^2$—CO—O— oder —O—CO— bedeuten und/oder R eine Carboxylgruppe enthält) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Nitrilen der Formel I (worin $A^1$ und/oder $A^2$ und/oder $A^3$ durch mindestens eine CN-Gruppe substituiert ist) ein entsprechendes Carbonsäureamid dehydratisiert oder ein entsprechendes Carbonsäurehalogenid mit Sulfamid umsetzt,

und/oder daß man gegebenenfalls eine Chlor- oder Bromverbindung der Formel I (worin $A^1$ und/oder $A^2$ und/oder $A^3$ durch mindestens ein Chlor- oder Bromatom substituiert ist) mit einem Cyanid umsetzt,

oder daß man zur Herstellung von Ethern der Formel I (worin R eine Alkoxygruppe bedeutet und/oder $Z^1$ und/oder $Z^2$ eine —$OCH_2$— oder —$CH_2O$-Gruppe ist) eine entsprechende Hydroxyverbindung verethert.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigelemente, die derartige Phasen enthalten.

Vor- und nachstehend haben R, $A^1$, $A^2$, $A^3$, $Z^1$, $Z^2$ und n die angegebene Bedeutung, sofern nicht ausdrücklich etwas anderes vermerkt ist.

Die Verbindungen der Formel I umfassen dementsprechend Verbindungen mit zwei Ringen der Teilformel I a:

$$R—A^2—Z^2—A^3—NCS \qquad I\,a$$

Verbindungen mit drei Ringen der Teilformeln I b und I c:

$$R—A^1—A^2—Z^2—A^3—NCS \qquad I\,b$$

$$R—A^1—Z^1—A^2—Z^2—A^3—NCS \qquad I\,c$$

sowie Verbindungen mit vier Ringen der Teilformeln I d bis I g:

$$R—A^1—A^1—A^2—Z^2—A^3—NCS \qquad I\,d$$

$$R—A^1—Z^1—A^1—A^2—Z^2—A^3—NCS \qquad I\,e$$

$$R—A^1—A^1—Z^1—A^2—Z^2—A^3—NCS \qquad I\,f$$

$$R—A^1—Z^1—A^1—Z^1—A^2—Z^2—A^3—NCS \qquad I\,g$$

sowie Verbindungen mit fünf Ringen der Teilformeln I h bis I o:

$$R—A^1—A^1—A^1—A^2—Z^2—A^3—NCS \qquad I\,h$$

$$R—A^1—Z^1—A^1—A^1—A^2—Z^2—A^3—NCS \qquad I\,i$$

$$R—A^1—A^1—Z^1—A^1—A^2—Z^2—A^3—NCS \qquad I\,j$$

$$R—A^1—A^1—A^1—Z^1—A^2—Z^2—A^3—NCS \qquad I\,k$$

$$R—A^1—Z^1—A^1—Z^1—A^1—A^2—Z^2—A^3—NCS \qquad I\,l$$

$$R—A^1—Z^1—A^1—A^1—Z^1—A^2—Z^2—A^3—NCS \qquad I\,m$$

$$R—A^1—A^1—Z^1—A^1—Z^1—A^2—Z^2—A^3—NCS \qquad I\,n$$

$$R—A^1—Z^1—A^1—Z^1—A^1—Z^1—A^2—Z^2—A^3—NCS \qquad I\,o$$

In den Verbindungen der vor- und nachstehend Formeln bedeutet R vorzugsweise Alkyl, ferner Alkoxy.

$A^1$, $A^2$ und $A^3$ sind bevorzugt Cy, Phe, Pym oder Pyr; be vorzugt enthalten die Verbindungen der Formel I nicht mehr als einen der Reste Bi, Pyd, Pym, Pyr oder Pyz.

$Z^1$ und $Z^2$ sind bevorzugt Einfachbindungen, in zweiter Linie bevorzugt —CO—O—, —O—CO— oder —$CH_2CH_2$-Gruppen.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (=Methoxymethyl), 2- (=Ethoxymethyl) oder 3-Oxabutyl (=2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine $CH_2$-Gruppe durch —CH=CH— ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formeln I mit verzweigten Flügelgruppen R können gelegentlich wegen einer besseren Löslichkeit in den üblichen flüssigkristallinen Basismaterialen von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste R sind Isopropyl, 2-Butyl (=1-Methylpropyl), Isobutyl (=2-Methylpropyl), 2-Methylbutyl, Isopentyl (=3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Formel I umfaßt sowohl die Racemate dieser Verbindungen als auch die optischen Antipoden sowie deren Gemische. Unter den Verbindungen der Formel I sowie I a bis I o sind diejenigen bevorzugt, in denen mindestens einer der darin enthaltenen Reste eine der angegebenen bevorzugten Bedeutungen hat.

Besonders bevorzugte kleinere Gruppen von Verbindungen sind diejenigen der Formel I 1 bis I 20, worin Phe 1,4-Phenylen, Cy 1,4-Cyclohexylen und PheF in 2- oder 3-Stellung durch Fluor substituiertes 1,4-Phenylen bedeutet:

| | |
|---|---|
| R—Cy—Cy—NCS | I 1 |
| R—Pym—Phe—NCS | I 2 |
| R—Pym—PheF—NCS | I 3 |
| R—Cy—COO—PheF—NCS | I 4 |
| R—Cy—Phe—Cy—NCS | I 5 |
| R—Cy—Pym—Phe—NCS | I 6 |
| R—Cy—$CH_2CH_2$—Phe—NCS | I 7 |
| R—Pym—$CH_2CH_2$—Phe—NCS | I 8 |
| R—Cy—$CH_2CH_2$—Cy—Phe—NCS | I 9 |
| R—Cy—$CH_2CH_2$—Cy—COO—Phe—NCS | I 10 |
| R—Cy—Phe—Phe—NCS | I 11 |
| R—Cy—PheF—Phe—NCS | I 12 |
| R—Cy—Phe—Phe—Cy—NCS | I 13 |
| R—Cy—PheF—Phe—Cy—NCS | I 14 |
| R—Pyr—Phe—NCS | I 15 |

R—Cy—Pyr—Phe—NCS I 16

R—Phe—Pyr—Phe—NCS I 17

R—Cy—Cy—Cy—Phe—NCS I 18

R—Cy—Cy—$CH_2CH_2$—Phe—NCS I 19

R—Cy—$CH_2CH_2$—Cy—Phe—NCS I 20

In den Verbindungen der Formel I sind diejenigen Stereoisomeren bevorzugt, in denen die Ringe Cy, Pip trans-1,4-disubstituiert sind. Diejenigen der vorstehend genannten Formeln, die eine oder mehrere Gruppen Pip, Pyd, Pym, Pyr, und/oder Pyz enthalten, umschließen jeweils die beiden 2,5- (Pym, Pyr) bzw. 1,4-Stellungsisomeren (Pip).

In den Verbindungen der Formel I, in denen $A^1$ für einen in 2-Stellung durch R substituierten Ring Pyd, Pym, Pyr oder Pyz steht, bedeutet R bevozugt Alkyl.

Besonders bevorzugt sind Verbindungen der Formel I worin R jeweils geradkettige oder höchstens einfach verzweigte Alkylgruppen oder Alkoxygruppen mit 1—12, insbesondere 2—10 C-Atomen bedeutet.

Besonders bevorzugt sind die folgenden kleineren Gruppen von Verbindungen, in denen Pym Pyrimidin-2,5-diyl, Phe 1,4-Phenylen, Cy 1,4-Cyclohexylen und PheF in 2- oder 3-Stellung durch Fluor substituiertes 1,4-Phenylen bedeutet.

Alkyl bedeutet vorzugsweise geradkettiges Methyl, Ethyl, Propyl, Butyl, Pentyl, Hexyl, Heptyl, Octyl, Nonyl oder Decyl; Oxaalkyl bedeutet vorzugsweise geradkettiges 2-Oxapropyl (=Methoxymethyl), 2-(=Ethoxymethyl) oder 3-Oxabutyl (=2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

I. Alkyl—Cy—Cy—NCS
Oxaalkyl—Cy—Cy—NCS

II. Alkyl—Pym—Phe—NCS
Alkoxycarbonyl—Pym—Phe—NCS
Alkyl—Pym—Phe—2F,NCS
Alkyl—Pym—Phe—3F,NCS
Alkoxycarbonyl—Pym—Phe—2F,NCS
Alkoxycarbonyl—Pym—Phe—3F,NCS

III. Alkyl—Cy—COO—Phe—2F,NCS
Alkyl—Cy—COO—Phe—3F,NCS

IV. Alkyl—Cy—Phe—Cy—NCS

V. Alkyl—Cy—Pym—Phe—NCS
Alkoxycarbonyl—Cy—Pym—Phe—NCS

VI. Alkyl—Pym—$CH_2CH_2$—Phe—NCS
Oxaalkyl—Pym—$CH_2CH_2$—Phe—NCS

VII. Alkyl—Cy—$CH_2CH_2$—Phe—NCS
Alkoxycarbonyl—Cy—$CH_2CH_2$—Phe—NCS

VIII. Alkyl—Cy—$CH_2CH_2$—Cy—Phe—NCS
Alkoxycarbonyl—Cy—$CH_2CH_2$—Cy—Phe—NCS
Alkyl—Cy—$CH_2CH_2$—Cy—Phe—2F,NCS
Alkyl—Cy—$CH_2CH_2$—Cy—Phe—3F,NCS
Alkoxycarbonyl—Cy—$CH_2CH_2$—Cy—Phe—2F,NCS
Alkoxycarbonyl—Cy—$CH_2CH_2$—Cy—Phe—3F,NCS

IX. Alkyl—Cy—$CH_2CH_2$—Cy—COO—Phe—NCS

X. Alkyl—Cy—Phe—Phe—NCS
Oxaalkyl—Cy—Phe—Phe—NCS
Alkyl—Cy—(Phe—2F)—Phe—NCS
Alkyl—Cy—(Phe—3F)—Phe—NCS

| | |
|---|---|
| XI. | Alkyl—Cy—Phe—Phe—Cy—NCS<br>Alkyl—Cy—(Phe—2F)—Cy—NCS<br>Alkyl—Cy—(Phe—3F)—Cy—NCS |
| XII. | Alkyl—Cy—Pyr—Phe—NCS<br>Alkyl—Phe—Pyr—Phe—NCS |
| XIII. | Alkyl—Pyr—Phe—NCS |
| XIV. | Alkyl—Cy—Cy—$CH_2CH_2$—Phe—NCS |
| XV. | Alkyl—Cy—Cy—Cy—Phe—NCS |

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C—C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanringes einen Cyclohexenring oder Cyclohexanonring und/oder an Stelle einer —$CH_2CH_2$—Gruppe eine —CH=CH—Gruppe und/oder an Stelle einer —$CH_2$—Gruppe eine —CO—Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder —$CH_2CH_2$—Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit $NaBH_4$ oder Tributylzinnhydrid in Methanol hydriert werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride. z.B. auch gemischte Anhydride, Azide oder Ester, insbesondere Alkylester mit 1—4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten

Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen −50° und +250°, vorzugsweise zwischen −20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin Collidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa −25° und +20°.

Zur Herstellung von Nitrilen der Formel I (worin $A^1$, $A^2$ und/oder $A^3$ durch mindestens eine CN-Gruppe substituiert ist) können entsprechende Säureamide, z.B. solche, in denen an Stelle des Restes CN eine $CONH_2$-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$(z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I (worin R eine Alkoxygruppe bedeutet und/oder worin $Z^1$ und/oder $Z^2$ eine $—OCH_2—$ oder eine $—CH_2O$-Gruppe ist) sind durch Veretherung entsprechender Hydroxyverbindungen, vorzugsweise entsprechender Phenole erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zur Herstellung von Nitrilen der Formel I (worin $A^1$, $A^2$ und/oder $A^3$ durch mindestens eine CN-Gruppe substituiert ist) können auch entsprechende Chlor- oder Bromverbindungen der Formel I (worin $A^1$ und/oder $A^2$ und/oder $A^3$ durch mindestens ein Cl- oder Br-Atom substituiert ist) mit einem Cyanid umgesetzt werden, zweckmäßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Verbindungen der Formel I, worin $A^1$, $A^2$ und/oder $A^3$ durch mindestens ein F- oder Cl-Atom und/oder eine CN-Gruppe substituiert ist, können auch aus den entsprechenden Diazoniumsalzen durch Austausch der Diazoniumgruppe gegen ein Fluor- oder Chloratom oder gegen eine CN-Gruppe, z.B. nach den Methoden von Schiemann oder Sandmeyer, erhalten werden.

Die Diazoniumsalze sind z.B. herstellbar durch Nitrierung von Verbindungen, die der Formel I entsprechen, aber an Stelle des Restes $NO_2$ ein Wasserstoffatom enthalten, Reduktion zu den entsprechenden Aminen und Diazotierung beispielsweise mit $NaNO_2$ oder $KNO_2$ in wässeriger Lösung bei Temperaturen zwischen etwa −10 und +10°.

Zum Austausch der Diazoniumgruppe gegen Fluor kann man in wasserfreier Flußsäure diazotieren und anschließend erwärmen, oder man setzt mit Tetrafluorborsäure zu den Diazoniumtetrafluorboraten um, die anschließend thermisch zersetzt werden.

Ein Austausch gegen Cl oder CN gelingt zweckmäßig durch Reaktion der wässerigen Diazoniumsalzlösung mit $Cu_2Cl_2$ oder $Cu_2(CN)_2$ nach der Methode von Sandmeyer.

Verbindungen der Formel I, worin $A^1$, $A^2$ und/oder $A^3$ durch mindestens eine $CF_3$-Gruppe substituiert ist, können aus Carbonsäuren oder deren Derivaten wie z.B. Säurehalogeniden oder -anhydriden mit Schwefeltetrafluorid bei erhöhter Temperatur erhalten werden.

Besonders vorteilhaft werden Verbindungen der Formel I durch Umsetzung von entsprechenden Aminen mit Thiophosgen erhalten, wie beispielsweise beschrieben in der deutschen Patentschrift 11 17 257.

Derartige Amine der Formel I, die anstelle der NCS-Gruppe eine Aminogruppe besitzen, sind bekannt oder können nach bekannten Methoden erhalten werden. So lassen sich Nitroverbindungen oder Oxime z.B. mit Wasserstoff unter Metallkatalyse oder mittels komplexer Hydride zu Aminoverbindungen reduzieren.

Auch können aliphatische oder aromatische Halogenverbindungen, wie beispielsweise Chloride, Bromide, und Iodide, mit Ammoniak oder einem Ammoniakäquivalent wie z.B. Phthalimidkalium unter Metallsalzkatalyse, insbesondere uner Zusatz von Kupfer und/oder seinen Salzen zu Aminen umgesetzt werden.

Die Reduktionspartner werden ohne Lösungsmittel oder zweckmäßig in Gegenwart eines inerten Lösungsmittels miteinander zur Reaktion gebracht.

Als Verdünnungsmittel eignen sich vorzugsweise Ether wie Diethylether, Dibutylether, Tetrahydrofuran, Dioxan, Amide wie Dimethylformamid, Hexamethylphosphorsäuretriamid, Kohlenwasserstoffe wie Hexan, Cyclohexan, Benzol, Toluol, Xylol, Halogenkohlenwasserstoffe wie Dichlormethan, Chloroform, Tetrachlorkohlenstoff, Tetrachlorethylen, Sulfoxide wie Dimethylsulfoxid, Sulfolan und weitere organische Lösungsmittel wie Acetonitril und Nitromethan. Auch Wasser oder Gemische dieser Lösungsmittel mit Wasser sind für die vorstehende Umsetzung geeignet. Die Reaktionstemperaturen liegen zwischen −20 und +100°, vorzugsweise zwischen 0 und 50°. Bei diesen Temperaturen sind die Umsetzungen in der Regel nach 30 Minuten bis 24 Stunden beendet.

In einem weiteren vorteilhaften Verfahren werden entsprechende Halogenide der Formel I, worin $A^3$ eine Cyclohexylengruppe bedeutet, mit einem Salz der Thiocyansäure umsetzt. Geeignete Salze hierfür sind beispielsweise Ammonium-, Natrium- und Kaliumrhodanid. Die Umsetzung wird vorteilhaft in Gegenwart eines inerten unpolaren Lösungsmittels durchgeführt. Gut geeignet sind insbesondere Kohlenwasserstoffe wie Hexan, Petrolether, Ligroin, Cyclohexan, Benzol, Toluol und Xylol. Die Reaktionstemperaturen liegen zwischen 0 und 150°, vorzugsweise zwischen 20 und 100°. Bei diesen Temperaturen sind die Umsetzungen in der Regel nach 1 bis 60 Stunden beendet.

Schließlich können auch Amine der Formel I, die anstelle der NCS-Gruppe eine Aminogruppe besitzen, mit Schwefelkohlenstoff in ein Dithiocarbamat überführt werden und diese weiter unter dem Einfluß verschiedener Reagenzien zu den erfindungsgemäßen Senfölen der Formel I umgesetzt werden. Zur Ausführung dieser Reaktionsfolge geeignete Reagenzien sind beispielsweise Wasserstoffperoxid (vgl. DE—21 05 473), Chlorcyan (DE—26 03 508), Cyanurchlorid (DE—19 35 302), Chlorkohlensäureester (DE—11 78 423), Alkalihypochlorit oder Alkalichlorit (DE 9 52 083), Phosgen (DE—10 68 250) und Schwermetallsalze (Dains et al., Org. Synth. Coll. Vol. 1 (1964) 447).

Die Herstellung der Dithiocarbamate aus den der Formel I entsprechenden Aminoverbindungen und Schwefelkohlenstoff erfolgt zweckmäßigerweise in einem inerten Lösungsmittel in Gegenwart eines Amins. Geeignete Lösungsmittel sind beispielweise Kohlenwasserstoffe wie Hexan, Petrolether, Ligroin, Cyclohexan, Benzol, Toluol und Xylol. Als Amin werden vorzugsweise tertiäre Amine wie beispielsweise Pyridin, Triethylamin oder Ethyldiisopropylamin verwendet. Die erhaltenen Ammoniumdithiocarbamate können durch z.B. Zentrifugieren oder Filtration isoliert werden, sie können jedoch auch in situ weiter zu Senfölen der Formel I umgesetzt werden.

Die Reaktionstemperaturen zur Herstellung der Dithiocarbamate betragen zwischen −20 und +100°, vorzugsweise zwischen 0 und 40°. Bei diesen Temperaturen sind die Umsetzungen in der Regel nach 1 bis 24 Stunden beendet.

Zur Umwandlung der Ammoniumdithiocarbamate wird vorzugsweise mit Chlorkohlensäureestern oder Wasserstoffperoxid umgesetzt.

Die isolierten oder in situ erhaltenen Ammoniumdithiocarbamate lassen sich, zweckmäßigerweise in Gegenwart eines geeigneten Lösungsmittels wie beispielsweise Ether wie Diethylether, Tetrahydrofuran, Dioxan und Halogenkohlenwasserstoffe wie Dichlormethan, Chloroform, Tetrachlorkohlenstoff, 1,2-Dichlorethan im Falle der Chlorkohlensäureester oder Wasser, Ameisensäure, Eissigsäure im Falle von Wasserstoffperoxid zur Reaktion bringen. Bei Reaktionstemperaturen von −20 bis 100°, vorzugsweise zwischen 0 und 80°, sind die Umsetzungen in der Regel nach 1 bis 24 Stunden beendet.

Insbesondere geeignet zur Herstellung von Senfölen der Formel I ist die Umsetzung geeigneter Amine mit Schwefelkohlenstoff und Triethylamin zu Triethylammonium-dithiocarbamaten und deren Überführung in Senföle durch Behandeln mit Chlorkohlensäurealkylester oder Wasserstoffperoxid.

Eine Base der Formel I kann mit einer Säure in das zugehörige Säureadditionssalz übergeführt werden. Für diese Umsetzung können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z.B. mit einer starken anorganischen Base wie KOH oder NaOH.

Die erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Biscyclohexylbenzole, 4,4'-Biscyclohexylbiphenyle, Phenyl- oder Cyclohexylpyrimidine, Phenyl- oder Cyclohexyldioxane, Phenyl- oder Cyclohexyldithiane, 1,2-Bis-phenylethane, 1,2-Biscyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

$$R^1—L—G—E—R^2 \qquad II$$

worin L und E je ein carbo- oder heterocyclisches Ring-system aus der aus 1,4-disubstituierten Benzol- und Cyclohexanringen, 4,4'-disubstituierten Biphenyl-, Phenylcyclohexan- und Cyclohexylcyclohexansystemen, 2,5-disubtituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | —CH=CH— | —N(O)=N— |
| | —CH=CY— | —CH=N(O)— |
| | —C≡C— | $—CH_2—CH_2—$ |
| | —CO—O— | $—CH_2—O—$ |
| | —CO—S— | $—CH_2—S—$ |
| | —CH=N— | —COO—Phe—COO— |

oder eine C—C-Einfachbindung, Y Halogen, vorzugsweise Chlor, oder —CN, und $R^1$ und $R^2$ Alkyl, Alkoxy, Alkanoyloxy oder Alkoxycarbonyloxy mit bis zu 18, vorzugsweise bis zu 8 Kohlenstoffatomen, oder einer dieser Reste auch CN, $NO_2$, $CF_3$, F, Cl oder Br bedeuten.

Bei den meisten dieser Verbindungen sind $R^1$ und $R^2$ voneinander verschieden, wobei einer dieser Reste meist eine Alkyl- oder Alkoxygruppe ist, Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen flüssigkristallinen Phasen enthalten etwa 0,1 bis 99, vorzugsweise 10 bis 95%, einer oder mehrerer Verbindungen der Formel I. Weiterhin bevorzugt sind flüssigkristalline Phasen, die 0,1—50, insbesondere 0,5—30% einer oder mehrerer Verbindungen der Formel I enthalten. Auch isotrope Verbindungen der Formel I können in den erfindungsgemäßen Phasen verwendet werden.

Die Herstellung der erfindungsgemäßen flüssigkristallinen Phasen erfolgt in an sich üblicher Weise. In der Regel werden die Komponenten ineinander gelöst, zweickmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst. Liq. Cryst. Band *24*, Seiten 249—258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE—OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben.

Beispiel 1

Zu einer Lösung von 15,2 g 1,1'-Thiocarbonyldiimidazol in 90 ml Dichlormethan wird innerhalb von 1,5 Stdn. bei 0° unter Stickstoff eine Lösung von 12,2 g trans - 4 - (4 - Aminocyclohexyl) - butylcyclohexan in 135 ml Dichlormethan zugetropft. Nach dem Stehen über Nacht wird bei 0—4° ca. 140 ml 3 %ige Salzsäure bis pH=2 zugesetzt. Die abgetrennte organische Phase wird mit Wasser bis pH=6 gewaschen, über Natriumsulfat getrocknet und eingedampft. Die säulenchromatographische Aufreinigung des Rohproduktes (15,6 g) ergibt 13,1 g trans - 4 - (4 - Isothiocyanatocyclohexyl) - butylcyclohexan. Schmp.+23°; Klp.+72,9°.

Analog hierzu werden hergestellt:

trans-4-(4-Isothiocyanatocyclohexyl)-ethylcyclohexan
trans-4-(4-Isothiocyanatocyclohexyl)-propylcyclohexan;
Schmp.+39°, Klp.+77,5°
trans-4-(4-Isothiocyanatocyclohexyl)-butylcyclohexan
trans-4-(4-Isothiocyanatocyclohexyl)-pentylcyclohexan
trans-4-(4-Isothiocyanatocyclohexyl)-hexylcyclohexan
trans-4-(4-Isothiocyanatocyclohexyl)-octylcyclohexan
trans-4-(4-Isothiocyanatocyclohexyl)-decylcyclohexan

Beispiel 2

38,3 g 4-(4-Aminocyclohexyl)-4'-(4-pentylcyclohexyl)biphenyl (erhalten aus 4,4'-Bis(4-oxocyclohexyl)-biphenyl durch Monoaddition von Amylmagnesiumbromid, Dehydratisierung des tertiären Alkohols und katalytische Hydrierung zu 4 - (4 - Pentylcyclohexyl) - 4' - (4 - oxocyclohexyl)biphenyl, dessen Überführung in das Oxim und Hydridreduktion) werden in 450 ml Dichlormethan und 15 ml Triethylamin gelöst und bei Raumtemperatur langsam mit 12 g Thiophosgen versetzt. Nach beendeter Zugabe läßt man noch 12 Stunden weiterrühren.

Zur Aufarbeitung wird mit Wasser gewaschen und die organische Phase vom Lösungsmittel befreit. Der Rückstand wird aus Ethanol kristallisiert, wodurch man 4 - (4 - Isothiocyanatocyclohexyl) - 4' - (pentylcyclohexyl) - biphenyl erhält.

Analog hierzu werden hergestellt:

4-(4-Isothiocyanatocyclohexyl)-4'-(4-propylcyclohexyl)-biphenyl
4-(4-Isothiocyanatocyclohexyl)-4'-(4-butylcyclohexyl)-biphenyl
4-(4-Isothiocyanatocyclohexyl)-4'-(4-hexylcyclohexyl)-biphenyl
4-(4-Isothiocyanatocyclohexyl)-4'-(4-heptylcyclohexyl)-biphenyl
4-(4-Isothiocyanatocyclohexyl)-4'-(4-octylcyclohexyl)-biphenyl
4-(4-Isothiocyanatocyclohexyl)-4'-(4-decylcyclohexyl)-biphenyl

Beispiel 3

48,6 g 4 - Amino - 4' - (4 - propylcyclohexyl)biphenyl (erhalten aus 4 - (4 - Propylcyclohexyl) - biphenyl durch Bromierung und Umsetzung des entsprechenden 4-Brombiphenyls mit Ammoniak/Wasser bei 200° in Gegenwart von Cu/CuCl) werden mit 10 g Schwefelkohlenstoff und 30 ml Triethylamin in 650 ml Toluol 30 Stunden bei 0° gerührt. Hiernach wird die abgeschiedene Kristallmasse des Triethylammoniumdithiocarbamats filtriert und mit Toluol gewaschen.

Man suspendiert das Zwischenprodukt in einem Gemisch aus 500 ml Chloroform und 50 ml Triethylamin. Anschließend wird tropfenweise Chlorkohlensäureethylester (21 g) zugegeben.

Zur Entfernung überschüssigen Triethylamins wäscht man mit verdünnter Salzsäure und dampft die Lösungsmittel unter vermindertem Druck ab. Nach Kristallisation des Rückstandes erhält man 4 - Isothiocyanato - 4' - (4 - propylcyclohexyl)biphenyl; Schmp. 149,3°, Klp. 240,5°.

Auf analoge Weise erhält man:

4-Isothiocyanato-4'-(4-butylcyclohexyl)-biphenyl;
Schmp. 119,8°, Klp. 233,0°
4-Isothiocyanato-4'-(4-pentylcyclohexyl)-biphenyl;
Schmp. 123,0°, Klp. 233,5°
4-Isothiocyanato-4'-(4-hexylcyclohexyl)-biphenyl;
Schmp. 96,2°, Klp. 223,0°
4-Isothiocyanato-4'-(4-heptylcyclohexyl)-biphenyl;
Schmp. 108,8°, Klp. 219,5°
4-Isothiocyanato-4'-(4-octylcyclohexyl)-biphenyl
4-Isothiocyanato-4'-(4-decylcyclohexyl)-biphenyl
4-Isothiocyanato-4'-(4-dodecylcyclohexyl)-biphenyl

Beispiel 4

29,1 g 4 - (4 - (4 - Pentylcyclohexyl)cyclohexyl) - anilin (erhalten aus 4 - (4 - (4 - Pentylcyclohexyl) - cyclohexyl)benzol analog der Herstellung von 4 - Amino - 4' - (4 - propylcyclohexyl)biphenyl) werden mit Schwefelkohlenstoff und Chlorkohlensäureethylester und Zwischenisolierung des Triethylammoniumdithiocarbamats in das entsprechende Senföl überführt.

Nach Kristallisation des Rohrproduktes erhält man (4 - (4 - (4 - Pentylcyclohexyl) - cyclohexyl) - phenylisothiocyanat.

Entsprechend werden erhalten:

4-(4-(4-Ethylcyclohexyl)-cyclohexyl)-phenylisothiocyanat

4-(4-(4-Propylcyclohexyl)-cyclohexyl)-phenylisothiocyanat
4-(4-(4-Butylcyclohexyl)-cyclohexyl)-phenylisothiocyanat
4-(4-(4-Hexylcyclohexyl)-cyclohexyl)-phenylisothiocyanat
4-(4-(4-Heptylcyclohexyl)-cyclohexyl)-phenylisothiocyanat
4-(4-(4-Octylcyclohexyl)-cyclohexyl)-phenylisothiocyanat
4-(4-(4-Nonylcyclohexyl)-cyclohexyl)-phenylisothiocyanat
4-(4-(4-Undecylcyclohexyl)-cyclohexyl)-phenylisothiocyanat

Beispiel 5

32,5 g 4 - (4 - (4 - Butylcyclohexylethyl) - cyclohexyl) - anilin (erhältlich aus 4 - (4 - (4 - Butylcyclohexylethyl) - cyclohexyl)benzol durch Bromierung und Ammonolyse wie in Beispiel 2 beschrieben) werden mit 12 g Thiophosgen unter Zusatz von 20 ml Triethylamin wie in Beispiel 3 beschrieben in das entsprechende Dithiocarbamat überführt.

Das Salz wird portionsweise in 150 ml 30%ige Wasserstoffperoxidlösung eingetragen und anschließend 2 Stunden bei 40° nachgerührt. Man läßt erkalten und saugt das ausgeschiedene Produkt ab. Nach Kristallisation aus Toluol wird 4 - (4 - (4 - Butylcyclohexylethyl) - cyclohexyl) - phenylsenföl erhalten.

Auf analoge Weise werden erhalten:

4-(4-(4-Propylcyclohexylethyl)-cyclohexyl)-phenylisothiocyanat
4-(4-(4-Pentylcyclohexylethyl)-cyclohexyl)-phenylisothiocyanat
4-(4-(4-Heptylcyclohexylethyl)-cyclohexyl)-phenylisothiocyanat
4-(4-(4-Octylcyclohexylethyl)-cyclohexyl)-phenylisothiocyanat
4-(4-(4-Nonylcyclohexylethyl)-cyclohexyl)-phenylisothiocyanat
4-(4-(4-Undecylcyclohexylethyl)-cyclohexyl)-phenylisothiocyanat
4-(4-(4-Tridecylcyclohexylethyl)-cyclohexyl)-phenylisothiocyanat

Beispiel 6

27,7 g 2 - Fluor-4-(4-heptylcyclohexyl) - anilin (erhältlich aus 2 - Fluor - 4 - bromnitrobenzol durch Lithiierung bei −100° und Umsetzung mit 4-Heptylcyclohexanon, Dehydratisierung des tertiären Alkohols und Reduktion von Doppelbindung und Nitrogruppe) werden mit 12 g Thiophosgen und 25 ml Triethylamin in 300 ml Chloroform 24 Stunden bei Raumtemperatur gerührt. Anschließend wird mit Wasser gewaschen und die organische Phase nach Trocknen eingeengt. Man erhält 2 - Fluor - 4 - (4 - heptylcyclohexyl) - phenylsenföl.

In analoger Weise werden erhalten:

2-Fluor-4-(4-propylcyclohexyl)-phenylisothiocyanat
2-Fluor-4-(4-butylcyclohexyl)-phenylisothiocyanat
2-Fluor-4-(4-pentylcyclohexyl)-phenylisothiocyanat
2-Fluor-4-(4-nonylcyclohexyl)-phenylisothiocyanat
2-Fluor-4-(4-decylcyclohexyl)-phenylisothiocyanat
2-Fluor-4-(4-dodecylcyclohexyl)-phenylisothiocyanat

Beispiel 7

2 - (4 - Aminophenyl) - 5 - pentylpyrimidin (hergestellt aus 4 - Aminophenylamidin und 2 - Pentylpropan - 1,3 - diol) wird mit Thiophosgen gemäß der in Beispiel 3 beschriebenen Weise umgesetzt. Man erhält 2 - (4 - Isothiocyanatophenyl) - 5 - pentylpyrimidin; Schmp. 46°, Klp. 85,5°.

Entsprechend werden erhalten:

2-(4-Isothiocyanatophenyl)-5-ethylpyrimidin
2-(4-Isothiocyanatophenyl)-5-propylpyrimidin
2-(4-Isothiocyanatophenyl)-5-butylpyrimidin;
Schmp. 60,8°, Klp. 77,8°
2-(4-Isothiocyanatophenyl)-5-hexylpyrimidin
2-(4-Isothiocyanatophenyl)-5-heptylpyrimidin
2-(4-Isothiocyanatophenyl)-5-octylpyrimidin
2-(4-Isothiocyanatophenyl)-5-nonylpyrimidin
2-(4-Isothiocyanatophenyl)-5-decylpyrimidin
2-(4-Isothiocyanatophenyl)-5-undecylpyrimidin
2-(4-Isothiocyanatophenyl)-5-dodecylpyrimidin
2-(4-Isothiocyanatophenyl)-5-tridecylpyrimidin

Beispiel 8

4 - Propylcyclohexancarbonsäure - (3 - fluor - 4 - aminophenyl)ester (erhältlich aus 4 - Propylcyclohexancarbonsäure und 3 - Fluor - 4 - nitro - phenol mittels Dicyclohexylcarbodiimid in Dichlormethan

sowie Anschließender Reduktion der Nitrogruppe mit Natriumdithionit) wird nach der in Beispiel 6 angegebenen Arbeitsweise zu 4-Propylcyclohexancarbonsäure - (3 - fluor - 4 - isothiocyanatophenyl) - ester umgesetzt.

Analog erhält man:

4-Butylcyclohexancarbonsäure-(3-fluor-4-isothiocyanatophenyl)-ester
4-Pentylcyclohexancarbonsäure-(3-fluor-4-isothiocyanatophenyl)-ester
4-Hexylcyclohexancarbonsäure-(3-fluor-4-isothiocyanatophenyl)-ester
4-Heptylcyclohexancarbonsäure-(3-fluor-4-isothiocyanatophenyl)-ester
4-Octylcyclohexancarbonsäure-(3-fluor-4-isothiocyanatophenyl)-ester
4-Nonylcyclohexancarbonsäure-(3-fluor-4-isothiocyanatophenyl)-ester
4-Decylcyclohexancarbonsäure-(3-fluor-4-isothiocyanatophenyl)-ester
4-Dodecylcyclohexancarbonsäure-(3-fluor-4-isothiocyanatophenyl)-ester

Beispiel 9

4 - (4 - Hexylcyclohexylethyl) - cyclohexancarbonsäure - (4 - aminophenyl) - ester (hergestellt aus 4 - (4 - Hexylcyclohexylethyl)cyclohexancarbonsäure und 4-Nitrophenol mittels Dicyclohexylcarbodiimid sowie anschließender katalytischer Reduktion der Nitrogruppe) wird nach der in Beispiel 6 angegebenen Arbeitsweise zu 4 - (4 - Hexylcyclohexylethyl) - cyclohexancarbonsäure(4 - isothiocyantophenyl) - ester umgesetzt.

Auf gleiche Weise werden erhalten:

4-(4-Propylcyclohexylethyl)-cyclohexancarbonsäure-(4-isothiocyanatophenyl)-ester
4-(4-Butylcyclohexylethyl)-cyclohexancarbonsäure-(4-isothiocyanatophenyl)-ester
4-(4-Pentylcyclohexylethyl)-cyclohexancarbonsäure-(4-isothiocyanatophenyl)-ester
4-(4-Heptylcyclohexylethyl)-cyclohexancarbonsäure-(4-isothiocyanatophenyl)-ester
4-(4-Octylcyclohexylethyl)-cyclohexancarbonsäure-(4-isothiocyanatophenyl)-ester
4-(4-Nonylcyclohexylethyl)-cyclohexancarbonsäure-(4-isothiocyanatophenyl)-ester
4-(4-Decylcyclohexylethyl)-cyclohexancarbonsäure-(4-isothiocyanatophenyl)-ester
4-(4-Undecylcyclohexylethyl)-cyclohexancarbonsäure-(4-isothiocyanatophenyl)-ester
4-(4-Dodecylcyclohexylethyl)-cyclohexancarbonsäure-(4-isothiocyanatopheny)-ester

Beispiel 10

26,8 g 2 - (4 - Aminophenyl) - 5 - heptylpyridin (hergestellt aus 5 - Heptyl - 2 - phenylpyridin durch Acylierung und Überführung des 2 - (4 - Acetylphenyl) - 5 - heptylpyridins in das entsprechende Oxim, dessen Beckmann-Umlagerung mittels Phosphortrichlorid/Pyridin und Hydrolyse des 2 - (4 - Acetamidophenyl) - 5 - heptylpyridins) werden mit Schwefelkohlenstoff und Chlorkohlensäureethylester auf die in Beispiel 3 beschriebene Weise umgesetzt.

Nach Kristallisation des Rohproduktes wird 4 - (5 - Heptylpyridin - 2 - yl) - phenylisothiocyanat erhalten.

Ensprechend werden erhalten:

4-(5-Ethylpyridin-2-yl-)-phenylisothiocyanat
4-(5-Propylpyridin-2-yl-)-phenylisothiocyanat
4-(5-Butylpyridin-2-yl-)-phenylisothiocyanat
4-(5-Hexylpyridin-2-yl-)-phenylisothiocyanat
4-(5-Octylpyridin-2-yl-)-phenylisothiocyanat
4-(5-Nonylpyridin-2-yl-)-phenylisothiocyanat
4-(5-Undecylpyridin-2-yl-)-phenylisothiocyanat
4-(5-(4-Ethylcyclohexyl)-pyridin-2-yl)-phenylisothiocyanat
4-(5-(4-Propylcyclohexyl)-pyridin-2-yl)-phenylisothiocyanat
4-(5-(4-Butylcyclohexyl)-pyridin-2-yl)-phenylisothiocyanat
4-(5-(4-Hexylcyclohexyl)-pyridin-2-yl)-phenylisothiocyanat
4-(5-(4-Heptylcyclohexyl)-pyridin-2-yl)-phenylisothiocyanat
4-(5-(4-Octylcyclohexyl)-pyridin-2-yl)-phenylisothiocyanat
4-(5-(4-Nonylcyclohexyl)-pyridin-2-yl)-phenylisothiocyanat
4-(5-(4-Undecylcyclohexyl)-pyridin-2-yl)-phenylisothiocyanat
4-(5-(4-Ethylphenyl)-pyridin-2-yl)-phenylisothiocyanat
4-(5-(4-Propylphenyl)-pyridin-2-yl)-phenylisothiocyanat
4-(5-(4-Butylphenyl)-pyridin-2-yl)-phenylisothiocyanat
4-(5-(4-Hexylphenyl)-pyridin-2-yl)-phenylisothiocyanat
4-(5-(4-Heptylphenyl)-pyridin-2-yl)-phenylisothiocyanat
4-(5-(4-Octylphenyl)-pyridin-2-yl)-phenylisothiocyanat
4-(5-(4-Nonylphenyl)-pyridin-2-yl)-phenylisothiocyanat
4-(5-(4-Undecylphenyl)-pyridin-2-yl)-phenylisothiocyanat

Beispiel 11

39,4 g 4 - (4 - (4 - (4 - Pentylcyclohexyl) - cyclohexyl) - cyclohexyl)-anilin (hergestellt aus (4 - (4 - (4 - Cyclohexyl) - cyclohexyl) - cyclohexyl) - benzol analog der im Beispiel 10 angegebenen Weise) werden mit Thiophosgen und Triethylamin in das entsprechende Dithiocarbamat überführt.

Nach Umsetzung mit 30%iger Wasserstoffperoxidlösung und Kristallisation des Rohprodukts erhält man 4 - (4 - (4 - (4 - Pentylcyclohexyl) - cyclohexyl) - cyclohexyl) - phenylisothiocyanat.

Auf analoge Weise werden erhalten:

4-(4-(4-(4-Ethylcyclohexyl)-cyclohexyl)-cyclohexyl)-phenylisothiocyanat
4-(4-(4-(4-Propylcyclohexyl)-cyclohexyl)-cyclohexyl)-phenylisothiocyanat
4-(4-(4-(4-Butylcyclohexyl)-cyclohexyl)-cyclohexyl)-phenylisothiocyanat
4-(4-(4-(4-Hexylcyclohexyl)-cyclohexyl)-cyclohexyl)-phenylisothiocyanat
4-(4-(4-(4-Heptylcyclohexyl)-cyclohexyl)-cyclohexyl)-phenylisothiocyanat
4-(4-(4-(4-Octylcyclohexyl)-cyclohexyl)-cyclohexyl)-phenylisothiocyanat
4-(4-(4-(4-Nonylcyclohexyl)-cyclohexyl)-cyclohexyl)-phenylisothiocyanat
4-(4-(4-(4-Undecylcyclohexyl)-cyclohexyl)-cyclohexyl)-phenylisothiocyanat

Beispiel 12

38,3 g 1 - (4 - (4 - Propylcyclohexyl) - cyclohexyl) - 2 - (4 - amino - phenyl) - ethan (erhalten aus (2 - (4 - (4 - Propylcyclohexyl) - cyclohexyl - ethyl) - benzol analog der in Beispiel 10 angegebenen Weise) werden mit 1,1' - Thiocarbonyldiimidazol in Dichlormethan gemäß dem in Beispiel 1 angewandten Verfahren zur Umsetzung gebracht.

Nach Kristallisation des Rohproduktes erhält man 1 - (4 - (4 - Propylcyclohexyl) - 2 - (4 - isothiocyanatophenyl) - ethan; Schmp. 43,0°, Klp. 38,6°.

Auf analoge Weise erhält man:

1-(4-(4-Ethylcyclohexyl)-2-(4-isothiocyanatophenyl)-ethan
1-(4-(4-Butylcyclohexyl)-2-(4-isothiocyanatophenyl)-ethan;
Schmp. 23,5°, Klp. 33,6°
1-(4-(4-Pentylcyclohexyl)-2-(4-isothiocyanatophenyl)-ethan;
Schmp. 41,0°, Klp. 47,5°
1-(4-(4-Hexylcyclohexyl)-2-(4-isothiocyanatophenyl)-ethan;
Schmp. 50,0°, Klp. 41,4°
1-(4-(4-Heptylcyclohexyl)-2-(4-isothiocyanatophenyl)-ethan;
Schmp. 56,2°, Klp. 49,2°
1-(4-(4-Octylcyclohexyl)-2-(4-isothiocyanatophenyl)-ethan
1-(4-(4-Nonylcyclohexyl)-2-(4-isothiocyanatophenyl)-ethan
1-(4-(4-Undecylcyclohexyl)-2-(4-isothiocyanatophenyl)-ethan

Beispiel 13

76,6 g 1 - (4 - Heptylcyclohexyl) - 2 - (4 - (4 - aminophenyl) - cyclohexyl) - ethan (hergestellt aus 1 - (Heptylcyclohexyl) - 2 - (4 - phenylcyclohexyl) - ethan analog der in Beispiel 10 angegebenen Weise) werden mit Thiophosgen und Triethylamin in das entsprechende Dithiocarbamat überführt.

Nach Umsetzung mit 30%iger Wasserstoffperoxidlösung und Kristallisation des Rohproduktes erhält man 1 - (4 - Heptylcyclohexyl) - 2 - (4 - (4 - isothiocyanatophenyl) - cyclohexyl) - ethan.

Auf analoge Weise werden erhalten:

1-(4-Propylcyclohexyl)-2-(4-(4-isothiocyanatophenyl)-cyclohexyl)-ethan
1-(4-Butylcyclohexyl)-2-(4-(4-isothiocyanatophenyl)-cyclohexyl)-ethan
1-(4-Pentylcyclohexyl)-2-(4-(4-isothiocyanatophenyl)-cyclohexyl)-ethan
1-(4-Hexylcyclohexyl)-2-(4-(4-isothiocyanatophenyl)-cyclohexyl)-ethan
1-(4-Octylcyclohexyl)-2-(4-(4-isothiocyantophenyl)-cyclohexyl)-ethan
1-(4-Nonylcyclohexyl)-2-(4-(4-isothiocyanatophenyl)-cyclohexyl)-ethan
1-(4-Decylcyclohexyl)-2-(4-(4-isothiocyanatophenyl)-cyclohexyl)-ethan
1-(4-Undecylcyclohexyl)-2-(4-(4-isothiocyanatophenyl)-cyclohexyl)-ethan
1-(4-Dodecylcyclohexyl)-2-(4-(4-isothiocyanatophenyl)-cyclohexyl)-ethan

**Patentansprüche**

1. Senföle der Formel I

$$R—(A^1—Z^1)_n—A^2—Z^2—A^3—NCS \qquad (I)$$

worin

R: H oder Alkyl mit 1—15 C-Atomen, worin auch eine oder zwei nicht benachbarte $CH_2$-Gruppen durch —CH=CH—, —O—, —CO—, —O—CO— und/oder —CO—O— ersetzt sein können,

$A^1$ und $A^2$ jeweils eine unsubstituierte oder ein- oder mehrfach substituierte 1,4-Cyclohexylengruppe, eine Piperidin-1,4-diyl- oder 1,4-Bicyclo-[2,2,2]octylengruppe oder eine unsubstituierte oder durch ein oder zwei F- und/oder Cl-Atome und/oder $CH_3$-Gruppen und/oder CN-Gruppen substituierte 1,4-Phenylengruppe, worin auch eine oder zwei CH-Gruppen durch N ersetzt sein können,

$A^3$: eine unsubstituierte oder ein- oder mehrfach substituierte 1,4-Cyclohexylengruppe oder eine unsubstituierte oder durch ein oder zwei F- und/oder Cl-Atome und/oder $CH_3$-Gruppen und/oder $CF_3$-Gruppen und/oder CN-Gruppen substituierte 1,4-Phenylengruppe,

$Z^1$ und $Z^2$: jeweils —CO—O—, —O—CO—, —O—, $—CH_2CH_2—$, $—CHCN—CH_2—$, $—CH_2—CHCN—$, —CH=CH—, $—OCH_2—$, $—CH_2O—$, —CH=N—, —N=CH—, —NO=N—, —N=NO— oder eine Einfachbindung,

n: 0, 1 oder 2 bedeutet,

mit der Maßgabe, daß, wenn $A^3$ eine unsubstituierte 1,4-Phenylengruppe bedeutet, n 2 ist oder $Z^2$ keine Einfachbindung oder $A^2—Z^2$ Pyr oder $Cy—CH_2CH_2—$, wobei Pyr eine Pyridin-2,5-diylgruppe und Cy eine 1,4-Cyclohexylengruppe ist, bedeutet und im Falle $Z^2$=—CO—O— $—(A^1—Z^1)_n—A^2—$ nicht

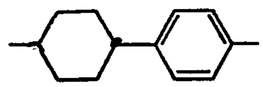

oder 1,4-Phenylen bedeutet.

2. Senföle nach Anspruch 1, dadurch gekennzeichnet, daß

$A^3$: eine 1,4-Cyclohexylgruppe oder eine durch ein Fluoratom substituierte 1,4-Phenylengruppe bedeutet.

3. Senföle nach Anspruch 1 oder 2 der Formeln I 1, I 3, I 7 und I 15:

| | |
|---|---|
| R—Cy—Cy—NCS | I 1 |
| R—Pym—PheF—NCS | I 3 |
| $R—Cy—CH_2CH_2—Phe—NCS$ | I 7 |
| R—Pyr—Phe—NCS | I 15 |

worin R die in Anspruch 1 gegebene Bedeutung besitzt und

| | |
|---|---|
| Cy | 1,4-Cyclohexylen |
| Pym | Pyrimidin-2,5-diyl |
| Phe | 1,4-Phenylen |
| PheF | 2-Fluor- oder 3-Fluor-1,4-phenylen, und |
| Pyr | Pyridin-2,5-diyl |

bedeutet.

4. Senföle nach Anspruch 1 der Formeln I 9, I 11 und I 19:

| | |
|---|---|
| $R—Cy—CH_2CH_2—Cy—Phe—NCS$ | I 9 |
| R—Cy—Phe—Phe—NCS | I 11 |
| $R—Cy—Cy—CH_2CH_2—Phe—NCS$ | I 19 |

worin R, Cy und Phe die angegebene Bedeutung besitzen.

5. Senföle nach Anspruch 1, 2 oder 3 ausgewählt aus der Gruppe

Alkyl—Cy—Cy—NCS

$Alkyl—Cy—CH_2CH_2—Phe—NCS$

Alkyl—Pyr—Phe—NCS

worin Cy, Phe, Pym und Pyr die angegebene Bedeutung besitzen und

Alkyl geradkettiges Methyl, Ethyl, Propyl, Butyl, Pentyl, Hexyl, Heptyl, Octyl, Nonyl oder Decyl bedeutet.

6. Senföle nach Anspruch 1 oder 4 ausgewählt aus der Gruppe:

Alkyl—Cy—$CH_2CH_2$—Cy—Phe—NCS

Alkyl—Cy—Phe—Phe—NCS

Alkyl—Cy—Cy—$CH_2CH_2$—Phe—NCS

worin Alkyl, Cy und Phe die angegebene Bedeutung besitzen.

7. Verfahren zur Herstellung von Senfölen der Formel I nach Anspruch 1, dadurch gekennzeichnet, daß man ein entsprechendes Amin mit Thiophosgen behandelt,

oder daß man ein entsprechendes Amin mit Schwefelkohlenstoff in ein Dithiocarbamat überführt und dieses mit Wasserstoffperoxid oder einem Chlorkohlensäureester behandelt,

oder daß man ein entsprechendes Halogenid der Formel I, worin $A^3$ eine Cyclohexylengruppe bedeutet, mit einem Salz der Thiocyansäure umsetzt,

oder daß man eine Verbindung, die sonst der Formel I entspricht, aber an Stelle von H-Atomen eine oder mehrere reduzierbare Gruppen und/oder C—C-Bindungen enthält, mit einem reduzierenden Mittel behandelt,

oder daß man zur Herstellung von Verbindungen der Formel I, worin $A^3$ eine durch ein oder zwei F- und/oder Cl-Atome und/oder CN-Gruppen substituierte 1,4-Phenylengruppe bedeutet, in einem entsprechenden Diazoniumsalz die Diazoniumgruppe durch F, Cl oder CN ersetzt,

oder daß man zur Herstellung von Benzotrifluoriden der Formel I, worin $A^3$ eine durch eine oder zwei $CF_3$-Gruppen substituierte 1,4-Phenylengruppe bedeutet, eine entsprechende Carbonsäure oder deren Anhydrid mit Schwefeltetrafluorid umsetzt,

oder daß man zur Herstellung von Estern der Formel I (worin $Z^1$ und/oder $Z^2$ —CO—O— oder —O—CO— bedeuten und/oder R eine Carboxylgruppe enthält) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Nitrilen der Formel I (worin $A^1$ und/oder $A^2$ und/oder $A^3$ durch mindestens eine CN-Gruppe substituierte ist) ein entsprechendes Carbonsäureamid dehydratisiert oder ein entsprechendes Carbonsäurehalogenid mit Sulfamid umsetzt,

und/oder daß man gegebenenfalls eine Chlor- oder Bromverbindung der Formel I (worin $A^1$ und/oder $A^2$ und/oder $A^3$ durch mindestens ein Chlor- oder Bromatom substituiert ist) mit einem Cyanid umsetzt,

oder daß man zur Herstellung von Ethern der Formel I (worin R eine Alkoxygruppe bedeutet und/oder $Z^1$ und/oder $Z^2$ eine —$OCH_2$— oder —$CH_2O$-Gruppe ist) eine entsprechende Hydroxyverbindung verethert.

8. Flüssigkristalline Phase mit mindestens zwei flüssigkristallinen Komponenten, dadurch gekennzeichnet, daß sie mindestens ein Senföl nach einen der Ansprüche 1 bis 6 enthält.

9. Flüssigkristallanzeigeelemente, dadurch gekennzeichnet, daß es eine flüssigkristalline Phase nach Anspruch 8 enthält.

10. Elektrisches Anzeigeelement, dadurch gekennzeichnet, daß es als Dielektrikum eine Phase nach Anspruch 8 enthält.

**Revendications**

1. Essences de moutarde de formule I

$$R—(A^1—Z^1)_n—A^2—Z^2—A^3—NCS \qquad (I)$$

où

R représente H ou un alkyle ayant 1 à 15 atomes de C dans lequel un ou deux groupes $CH_2$ non voisins peuvent être remplacées par —CH=CH—, —O—, —CO—, —O—CO— et/ou —CO—O—,

$A^1$ et $A^2$ représentent un groupe 1,4-cyclohexylène non substitué ou une ou plusieurs fois substitué, un groupe pipéridin-1,4-diyle ou 1,4-bicyclo[2,2,2]octylène ou un groupe 1,4-phénylène non substitué ou substitué par un ou deux atomes de F et/ou de Cl et/ou par des groupes $CH_3$ et/ou par des groupes CN où également un ou deux groupes CH peuvent être remplacés par N,

$A^3$ représente un groupe 1,4-cyclohexylène non substitué ou une ou plusieurs fois substitué ou un groupe 1,4-phénylène non substitué ou substitué par un ou deux atomes de F et/ou de Cl et/ou par des groupes $CH_3$ et/ou par des groupes $CF_3$ et/ou par des groupes CN,

$Z^1$ et $Z^2$ représentent —CO—O—, —O—CO—, —O—, —$CH_2CH_2$—, —CHCN—$CH_2$—, —$CH_2$—CHCN—, —CH=CH—, —$OCH_2$—, —$CH_2O$—, —CH=N—, —N=CH—, —NO=N—, —N=NO— ou une liaison simple,

n égal 0, 1 ou 2

sous réserve que, lorsque $A^3$ représente un groupe 1,4-phénylène non substitué, n est égal à 2 ou $Z^2$ ne représente pas une liaison simple ou $A^2$—$Z^2$ représente Pyr ou Cy—$CH_2CH_2$—, Pyr étant un groupe pyridin-2,5-diyle et Cy un groupe 1,4-cyclohexylène, et dans le cas où $Z^2$=—CO—O—, —$(A^1—Z^1)_n$—$A^2$— ne représente pas

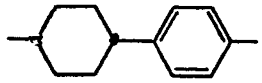

ou le 1,4-phénylène.

2. Essences de moutarde selon la revendication 1, caractérisées en ce que $A^3$ représente un groupe 1,4-cyclohexylène ou un groupe 1,4-phenylène substitué par un atome de fluor.

3. Essences de moutarde selon la revendication 1 ou 2 de formules I 1, I 3, I 7 et I 15:

| | |
|---|---|
| R—Cy—Cy—NCS | I 1 |
| R—Pym—PheF—NCS | I 3 |
| R—Cy—$CH_2CH_2$—Phe—NCS | I 7 |
| R—Pyr—Phe—NCS | I 15 |

où R a la signification donnée dans la revendication 1 et

| | |
|---|---|
| Cy | représente le 1,4-cyclohexylène |
| Pym | la pyrimidin-2,5-diyle |
| Phe | le 1,4-phénylène |
| PheF | le 2-fluoro- ou le 3-fluoro-1,4-phénylène et |
| Pyr | la pyridin-2,5-diyle. |

4. Essences de moutarde selon la revendication 1 de formules I 9, I 11 et I 19:

| | |
|---|---|
| R—Cy—$CH_2CH_2$—Cy—Phe—NCS | I 9 |
| R—Cy—Phe—Phe—NCS | I 11 |
| R—Cy—Cy—$CH_2CH_2$—Phe—NCS | I 19 |

où R, Cy et PHe ont la signification indiquée.

5. Essences de moutarde selon la revendication 1, 2 ou 3 choisies dans le groupe

alkyl—Cy—Cy—NCS

alkyl—Cy—$CH_2CH_2$—Phe—NCS

alkyl—Pyr—Phe—NCS

où Cy, Phe, Pym et Pyr ont la signification indiquée et où alkyle signifie le méthyle, l'éthyle, le propyle, le butyle, le pentyle, l'hexyle, l'heptyle, l'octyle, le nonyle ou le décyle linéaires.

6. Essences de moutarde selon la revendication 1 ou 4 choisies dans le groupe

alkyl-Cy-$CH_2CH_2$—Cy—Phe—NCS

alkyl—Cy—Phe—Phe—NCS

alkyl—Cy—Cy—$CH_2CH_2$—Phe—NCS

où alkyle, Cy et Phe ont la signification indiquée.

7. Procédé pour la préparation d'essences de moutarde de formule I selon la revendication 1, caractérisé en ce que l'on traite une amine correspondante avec le thiophosgène ou en ce que l'on transforme une amine correspondante avec le sulfure de carbone en un dithiocarbamate et en ce que l'on traite celui-ci avec le l'eau oxygénée ou un ester de l'acide chlorocarbonique ou en ce que l'on fait réagir un halogénure correspondant de formule I où $A^3$ représente un groupe cyclohexylène avec un sel de l'acide thiocyanique

ou en ce que l'on traite un composé qui correspond à la formule I, mais qui à la place d'atomes de H contient un ou plusieurs groupes réductibles et/ou des liaisons C—C, avec un agent réducteur,

ou en ce que, pour la préparation des composés de formule I où $A^3$ représente un groupe 1,4-phénylène substitué par un ou deux atomes de F et/ou de Cl et/ou des groupes CN, on remplace dans un sel de diazonium correspondant le groupe diazonium par F, Cl ou CN,

ou en ce que, pour la préparation des benzotrifluorures de formule I où $A^3$ représente un groupe 1,4-phenylène substitué par un ou deux groupes $CF_3$, on fait réagir un acide carboxylique correspondant ou son anhydride avec du tétrafluorure de soufre,

ou en ce que, pour la préparation des esters de formule I (où $Z^1$ et/ou $Z^2$ représentent —CO—O— ou —O—CO— et/ou R contient un groupe carboxyle), on fait réagir un acide carboxylique correspondant ou l'une de ses dérivés réactifs avec un alcool correspondant ou l'un de ses dérivés réactifs,

ou en ce que, pour la préparation des nitriles de formule I (où $A^1$ et/ou $A^2$ et/ou $A^3$ sont substitués par au moins un groupe CN), on déshydrate un amide d'acide carboxylique correspondant ou on fait réagir un halogénure d'acide carboxylique correspondant avec un sulfamide et/ou en ce que l'on fait réagir éventuellement un composé chloré ou bromé de formule I (où $A^1$ et/ou $A^2$ et/ou $A^3$ sont substitués par au moins un atome de chlore ou de brome) avec un cyanure

ou en ce que, pour la préparation des éthers de formule I (où R représente un groupe alcoxy et/ou $Z^1$ et/ou $Z^2$ est un groupe $—OCH_2$ ou $—CH_2O$), on éthérifie un composé hydroxylé correspondant.

8. Phase à cristaux liquides contenant au moins deux composants à cristaux liquides, caractérisée en ce qu'elle contient au moins une essence de moutarde selon l'une des revendications 1 à 6.

9. Elément d'affichage à cristaux liquides, caractérisé en ce qu'il contient une phase à cristaux liquides selon la revendication 8.

10. Elément d'affichage électro-optique, caractérisé en ce qu'il contient comme diélectrique une phase selon la revendication 8.

**Claims**

1. Mustard oils of the formula I

$$R—(A^1—Z^1)_n—A^2—Z^2—A^3—NCS \qquad (I)$$

in which

R is H or alkyl of 1—15 C atoms, in which one or two non-adjacent $CH_2$ groups can be replaced by —CH=CH—, —O—, —CO—, —O—CO— and/or —CO—O—,

$A^1$ and $A^2$ are each an unsubstituted or monosubstituted or polysubstituted 1,4-cyclohexylene group, a piperidine-1,4-diyl or 1,4-bicyclo[2,2,2]octylene group or a 1,4-phenylene group which is unsubstituted or substituted by one or two F and/or Cl atoms and/or $CH_3$ groups and/or CN groups and in which one or two CH groups can also be replaced by N,

$A^3$ is an unsubstituted or monosubstituted or polysubstituted 1,4-cyclohexylene group or a 1,4-phenylene group which is unsubstituted or substituted by one or two F and/or Cl atoms and/or $CH_3$ groups and/or $CF_3$ groups and/or CN groups,

$Z^1$ and $Z^2$ are each —CO—O—, —O—CO—, —O—, $—CH_2CH_2—$, $—CHCN—CH_2—$, $—CH_2—CHCN—$, —CH=CH—, $—OCH_2—$, $—CH_2O—$, —CH=N—, —N=CH—, —NO=N—, —N=NO— or a single bond,

n is 0, 1 or 2,

with the proviso that, when $A^3$ is an unsubstituted 1,4-phenylene group, n is 2 or $Z^2$ is no single bond or $A^2—Z^2$ is Pyr or $Cy—CH_2CH_2—$, where Pyr is a pyridine-2,5-diyl group and Cy is a 1,4-cyclohexylene group, and in the case of $Z^2$=—CO—O—, $—(A^1—Z^1)_n—A^2—$ is not

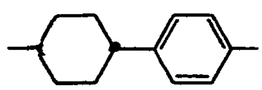

or 1,4-phenylene.

2. Mustard oils according to Claim 1, characterized in that

$A^3$ is a 1,4-cyclohexylene group or a fluorine-substituted 1,4-phenylene group.

3. Mustard oils according to Claim 1 or 2 of the formulae I 1, I 3, I 7 and I 15:

| | |
|---|---|
| R—Cy—Cy—NCS | I 1 |
| R—Pym—PheF—NCS | I 3 |
| $R—Cy—CH_2CH_2—Phe—NCS$ | I 7 |
| R—Pyr—Phe—NCS | I 15 |

in which R is as defined in Claim 1 and

Cy is 1,4-cyclohexylene
Pym is pyrimidine-2,5-diyl
Phe is 1,4-phenylene
PheF is 2-fluoro- or 3-fluoro-1,4-phenylene, and
Pyr is pyridine-2,5-diyl.

4. Mustard oils according to Claim 1 of the formulae I 9, I 11 and I 19:

$$R—Cy—CH_2CH_2—Cy—Phe—NCS \quad I\ 9$$

$$R—Cy—Phe—Phe—NCS \quad I\ 11$$

$$R—Cy—Cy—CH_2CH_2—Phe—NCS \quad I\ 19$$

in which R, Cy and Phe are as defined above.

5. Mustard oils according to Claim 1, 2 or 3 selected from the group

Alkyl—Cy—Cy—NCS

Alkyl—Cy—$CH_2CH_2$—Phe—NCS

Alkyl—Pyr—Phe—NCS

in which Cy, Phe, Pym and Pyr are as defined above and
Alkyl is straight-chain methyl, ethyl, propyl, butyl, pentyl, hexyl, heptyl, octyl, nonyl or decyl.

6. Mustard oils according to Claim 1 or 4 selected from the group:

Alkyl—Cy—$CH_2CH_2$—Cy—Phe—NCS

Alkyl—Cy—Phe—Phe—NCS

Alkyl—Cy—Cy—$CH_2CH_2$—Phe—NCS

in which Alkyl, Cy and Phe are as defined above.

7. Process for preparing mustard oils of the formula I according to Claim 1, characterized in that an appropriate amine is treated with thiophosgene, or in that an appropriate amine is converted with carbon disulfide into a dithiocarbamate and the latter is treated with hydrogen peroxide or a chlorocarbonic acid ester,

or in that an appropriate halide of the formula I in which $A^3$ is a cyclohexylene group is reacted with a salt of thiocyanic acid,

or in that a compound which otherwise conforms to the formula I but contains one or more reducible groups and/or C—C bonds in place of H atoms is treated with a reducing agent,

or in that, to prepare compounds of the formula I in which $A^3$ is a 1,4-phenylene group which is substituted by one or two F and/or Cl atoms and/or CN groups, the diazonium group in an appropriate diazonium salt is replaced by F, Cl or CN,

or in that, to prepare benzotrifluorides of the formula I in which $A^3$ is a 1,4-phenylene group which is substituted by one or two $CF_3$ groups, an appropriate carboxylic acid or the anhydride thereof is reacted with sulfur tetrafluoride,

or in that, to prepare esters of the formula I (in which $Z^1$ and/or $Z^2$ are —CO—O— or —O—CO— and/or R contains a carboxyl group), an appropriate carboxylic acid or one of the reactive derivatives thereof is reacted with an appropriate alcohol or a reactive derivative thereof,

or in that, to prepare nitriles of the formula I (in which $A^1$ and/or $A^2$ and/or $A^3$ is substituted by at least one CN group), an appropriate carboxamide is dehydrated or an appropriate carbonyl halide is reacted with sulfamide,

and/or in that if desired a chlorine or bromine compound of the formula I (in which $A^1$ and/or $A^2$ and/or $A^3$ is substituted by at least one chlorine or bromine atom) is reacted with a cyanide,

or in that, to prepare ethers of the formula I (in which R is an alkoxy group and/or $Z^1$ and/or $Z^2$ is an —$OCH_2$— or —$CH_2O$— group), an appropriate hydroxy compound is etherified.

8. Liquid-crystalline phase having at least two liquid-crystalline components, characterized in that it contains at least one mustard oil according to any one of Claims 1 to 6.

9. Liquid-crystal display element, characterized in that it contains a liquid-crystalline phase according to Claim 8.

10. Electro-optical display element, characterized in that it contains as dielectric a phase according to Claim 8.